(19) Europäisches Patentamt
European Patent Office
Office européen des brevets

(11) Publication number: **0 126 813**
**B1**

(12) # EUROPEAN PATENT SPECIFICATION

(45) Date of publication of patent specification: **02.01.91**

(21) Application number: **83302986.1**

(22) Date of filing: **24.05.83**

(51) Int. Cl.[5]: **C 07 D 233/90,**
C 07 D 473/18,
C 07 D 473/30,
A 61 K 31/415, A 61 K 31/52
// C07D475/02, C07D241/06

(54) **Process for preparing imidazole compounds.**

(43) Date of publication of application:
**05.12.84 Bulletin 84/49**

(45) Publication of the grant of the patent:
**02.01.91 Bulletin 91/01**

(84) Designated Contracting States:
**AT BE CH DE FR GB IT LI LU NL SE**

(56) References cited:
**EP-A-0 036 077**
**EP-A-0 055 239**
**EP-A-0 066 909**
**EP-A-0 072 027**
**US-A-4 221 909**

**JOURNAL OF THE CHEMICAL SOCIETY. PERKIN I., Nr. 17, 1976, W.F. KEIR et al. "Amidinoacetamides in the synthesis of pyrimidines, imidazoles and purines", pages 1847-1852**

**COMPREHENSIVE HETEROCYCLIC CHEMISTRY, Vol. 5, Part 4A, Pergamon Press, pages 583-585**
**An Introduction to the Chemistry and Biochemistry of Pyrimidines, Purines and Pteridines, John Wiley & Sons, pages 71,72**

(73) Proprietor: **Newport Pharmaceuticals International, Inc.**
**897 West 16th Street**
**Newport Beach California (US)**

(72) Inventor: **Simon, Lionel Norton**
**11772 Las Palmas**
**Santa Ana California 92709 (US)**
Inventor: **Mueller, Hans-Rudolf**
**Beckenwaildli 18**
**CH-8207 Schaffhausen (CH)**
Inventor: **Zutter, Hans**
**Stauffacherstrasse 19**
**CH-8200 Schaffhausen (CH)**

(74) Representative: **De Minvielle-Devaux, Ian Benedict Peter et al**
**CARPMAELS & RANSFORD**
**43, Bloomsbury Square**
**London WC1A 2RA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 126 813 B1

**Description**

Simon, U.S. patents 4,221,909 and 4,211,794, and Giner-Sorolla, U.S. patent 4,221,910, and Faanes, International Journal of Imunopharmacology, Vol. 2, No. 3, page 197 (1980), Florentine, International Journal of Immunpharmacology, Vol. 2, No. 3, page 240 (1980), Hadden, International Journal of Immunopharmacology, Vol. 2, No. 3, page 198 (1980), Pahwa, International Journal of Immunopharmacology, Vol. 2, No. 3, page 199 (1980), Wybran, Internatiaonal Journal of Immunopharmacology, Vol. 2, No. 3, page 201 (1980) and Simon, 4th International Congress of Immunology, Paris (1980), show that erythro-9-(2-hydroxy-3-nonyl)-hydroxanthine (NPT 15392), as well as other members of the series described in the patents cited above are potent immunomodulating agents which have been demonstrated to enhance depressed immunity in both animals possessing tumors, Sato, International Journal of Immunopharmacology, Vol. 2, No. 3, page 200 (1980), as well as in humans with various tumors, Simon, American Chemical Society Book of Abstracts, 182nd American Chemical Society Meeting (1981). While the synthesis of these therapeutically useful agents can be carried out by the methods described in the patents cited above, their production, e.g. the production of NPT 15392, on a large scale using those methods was cumbersome, costly, and time consuming.

The present invention provides a process of preparing a compound of the general formula 1

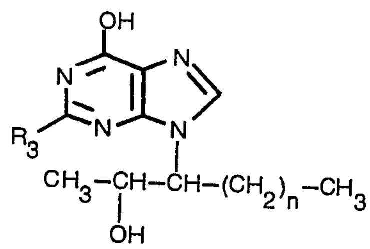

(1)

wherein

$R_3$ is hydrogen or lower alkyl and

n is an integer from 1 to 5, comprising reacting a compound of the general formula 2

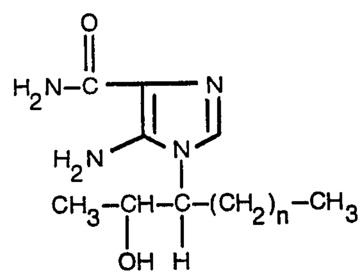

(2)

with a lower alkyl orthoester of a lower fatty acid or with formic acid in the presence of acetic anhydride.

The present invention also provides a process of preparing a compound of the general formula 3

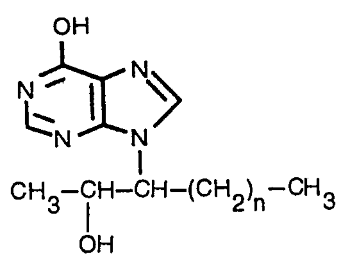

(3)

in which

n is an integer from 1 to 5, comprising reacting a compound of the general formula 2 above with an equimolar amount of a lower alkyl orthoformate or with formic acid in the presence of acetic anhydride.

Examples of lower alkyl groups are those with 1 to 4 carbon atoms, such as methyl, ethyl, propyl, or butyl.

The compound erythro-3-(2-hydroxy-3-nonyl)-4-amino-imidazole-5-carboxamide (NPT 15459) is useful in producing NPT 15392 and its homologues of general formula 2 above are useful in making the corresponding homologues of NPT 15392. In addition to being useful in making NPT 15392 and its homologues, NPT 15459 and its homologues are useful in preparing analogues of NPT 15392 (and its homologues) which contain lower alkyl groups in the 2-position of the purine ring. Such derivatives would be difficult to prepare without the aid of NPT 15459 and its homologues.

The compounds of general formulae 1, 2 and 3 also have immunomodulating or immunopotentiating activity.

An immunopotentiator or immunomodulator is any agent which either restores depressed immune function, or enhances normal immune function, or both. Immune function is defined as the development and expression of humoral (antibody-mediated) immunity, cellular (thymocyte-mediated) immunity, or macrophage and granulocyte mediated resistance. It logically includes agents acting directly on the cells involved in the expression of immune response, or on celular or molecular mechanisms which, in turn, act to modify the function of cells involved in immune response. Augmentation of immune function may result from the action of an agent to abrogate suppressive mechanisms derived by negative-feedback influences endogenous or exogenous to the immune system. Thus, immune potentiators have diverse mechanisms of action. Despite the diversity of cell site of action and biochemical mechanism of action of immunopotentiators, their applications are essentially the same; that is, to enhance host resistance.

Applications of Immunopotentiators

1) The principal protective function of the immune system relates to resistance to invasion by pathogens, including viruses, rickettsia, mycoplasma, bacteria, fungi and parasites of all types. Thus, improvement of immune response, particularly when depressed, would calculatedly improve resistance in infection or infestation by any of the above pathogens. An immunopotentiator alone or in combination with anti-infective therapy can be applied to any and all infectious diseases.

2) A second protective function of the immune system is thought to be resistance to engraftment of foreign tissue, either natural as in the fetal-maternal relationship; or unnatural as performed by the transplant physician. Immunopotentiators can also be used to facilitate rejection of fetal or placental tissues or to modify or induce tolerance to grafts.

3) A third protective function of the immune system is thought to be resistance to malignant cell development as in cancer. The use of immunopotentiators can be used in cancer treatment to enhance tumor rejection and to inhibit tumor recurrences following other forms of therapy.

4) A fourth protective function involves the capacity to recognize foreignness and to maintain nonreactivity to self by positive suppressor mechanisms. In auto-immune and related disorders, immune reactivity directed at self antigens or exaggerated, elevated responses are apparent which are self-destructive. Immunopotentiators can be used to restore normal suppressor mechanisms, induce tolerance, or otherwise promote a normal immune response.

Each of the protective functions of the immune system can be modified by non-specific therapy with immunopotentiators alone or in combination with other agents employed to improve resistance or to kill the invading pathogen. In addition, specific resistance can be augmented by use of immunopotentiators in conjuction with some form of antigen as in a vaccine employing, for example, virus or tumor cells. This use can be to induce either specific immunity or tolerance. The latter might be exemplified by use with antigen in allergy or auto-immune diseases. Use of immunopotentiators may be either therapeutic or prophylactic; the latter particularly in aging, where infection, auto-immunity, and cancer are more common. The timing of administration and routes are variable and may be critical in determining whether a positive or negative response results. Any agent capable of augmenting immune response may inhibit it depending on timing and dose, thus, under cetain circumstances an immunopotentiator could be used as an immunosuppressive agent for use in allergy, auto-immunity and transplantation.

Various procedures for determining immunomodulating activity are shown in EP—A—0036077 (Simon).

Brief Description of the Drawings

Figure 1 is the IR-spectrum of NPT 15459, and
Figure 2 is the UV-spectrum of NPT 15459.

Description of Preferred Embodiments

Illustative of compounds within the general formula 2, in addition of NPT 15459, are:
erythro-3-(2-hydroxy-3-amyl)-4-amino-imidazole-5-carboxamide,
erythro-3-(2-hydroxy-3-hexyl)-4-amino-imidazole-5-carboxamide,
erythro-3-(2-hydroxy-3-heptyl)-4-amino-imidazole-5-carboxamide, and
erythro-3-(2-hydroxy-3-octyl)-4-amino-imidazole-5-carboxamide.

Illustrative of compounds within the general formula 1, in addition to NPT 15392, are:
erythro-9-(2-hydroxy-3-nonyl)-2-methyl-hypoxanthine,
erythro-9-(2-hydroxy-3-amyl)-2-methyl-hypoxanthine,
erythro-9-(2-hydroxy-3-hexyl)-2-methyl-hypoxanthine,
erythro-9-(2-hydroxy-3-heptyl)-2-methyl-hypoxanthine,
erythro-9-(2-hydroxy-3-octyl)-2-methyl-hypoxanthine,
erythro-9-(2-hydroxy-3-nonyl)-2-ethyl-hypoxanthine,
erythro-9-(2-hydroxy-3-hexyl)-2-ethyl-hypoxanthine, and
erythro-9-(2-hydroxy-3-amyl)-2-ethyl-hypoxanthine.

The synthesis of NPT 15459 is carried out according to the scheme presented below:

(1)

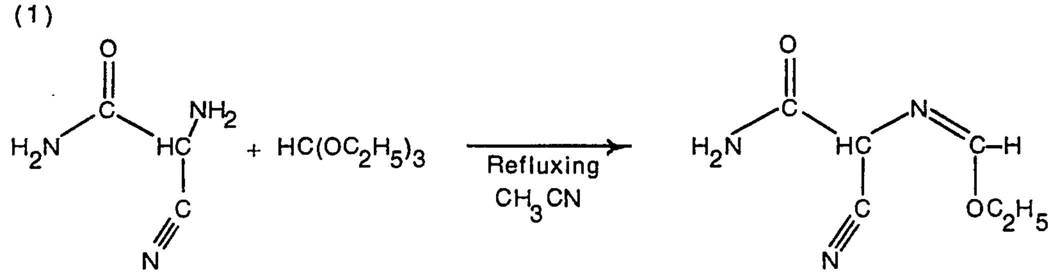


(I) (II) (III)

(2)

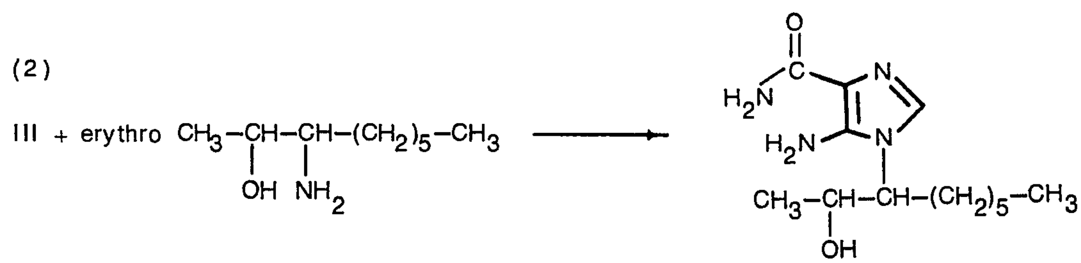

(IV)

(V) (NPT 15459)

The other compounds within general formula 1 can be prepared in the same manner by replacing erythro-3-amino-2-nonanol by the corresponding aminoalcohol, e.g. erythro-3-amino-2-pentanol and erythro-3-amino-2-octanol. In place of ethyl orthoformate there can be used other lower alkyl orthoformates, e.g. methyl orthoformate, propyl orthoformate and butyl orthoformate.

Example 1

Synthesis of Erythro-3-(2-hydroxy-3-nonyl)-4-amino-imidazole-5-carboxamide (NPT 15459 (V))

For improved yield, reactions (1) and (2) above are carried out sequentially without isolation of the intermediate product III. 2-Amino-2-cyano-acetamide (I) (171 g, 1.71 moles) is suspended in 2200 ml of acetonitrile. Orthoformic acid triethylester (II) (334 ml — 2.0 moles) and 2 ml of pyridine are added to the suspension with stirring. The suspension is heated to reflux temperature, using an oil bath preheated to 100°C. The suspension is held at boiling temperature for 40 to 60 minutes. III is produced in situ. 272.5 g of erythro-3-amino-2-nonanol (IV) (1.71 moles) are then added over a 3 to 5 minute period and boiling is continued for an additional 10 to 15 minutes. The reaction is quickly chilled to room temperature. The erythro-3-(2-hydroxy-3-nonyl)-4-amino-imidazole-5-carboxamide (NPT 15459) (V) crystallizes. It is filtered by suction, washed with a small amount of acetonitrile and dried in vacuo at 70°C.

Yield: 330.4 g, 72% of theory.

Melting point (after recrystallization from acetonitrile): 154—158°C.

Summary of Chemical Properties of (NPT 15459)

| Chemical or Physical Property | Value | |
|---|---|---|
| M.P. | 154—158°C | |
| C% | Theory: 15.18 | Found: 57.80 |
| N% | Theory: 20.87 | Found: 21.00 |
| Solubility | — soluble-isopropanol<br>— very soluble — HCl (Aq), acetic acid methanol, ethanol<br>— insoluble — benzene, water | |
| TLC | silica gel (ethylacetate/methanol: 8:2) Rf = 0.61 | |
| Mol. Wt. | 268.35 | |

Appearance: Colorless and odorless powder

Identity

1. IR-spectrum: (Fig. 1) —

| | | | |
|---|---|---|---|
| bands at | $3450\ cm^{-1}$ | | $(-NH_2)$ |
| | $3400-3100\ cm^{-1}$ | (several) | $(-CONH_2$ a.o.) |
| | $2950\ cm^{-1}$ | | $(-CH_3-, -CH_2-)$ |
| | $1660\ cm^{-1}$ | | (-imidazole) |

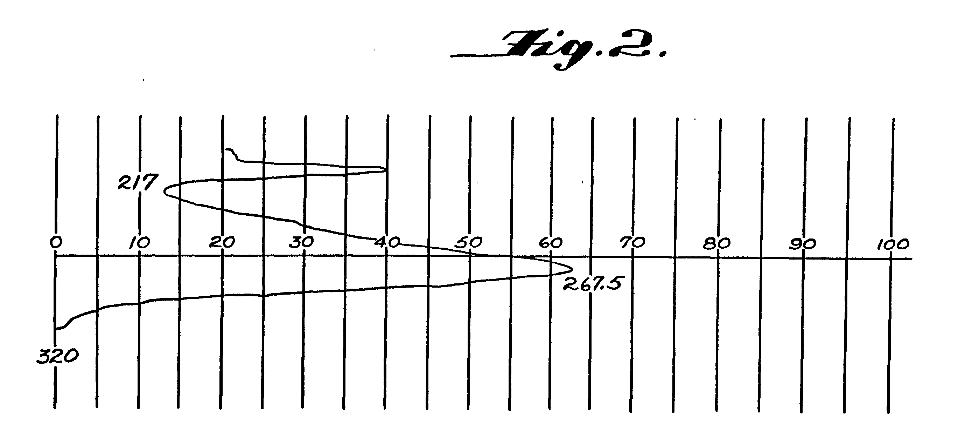

2. UV-spectrum: (Fig. 2) — maximum at 267 nm
maximum at 217 nm

| | | |
|---|---|---|
| Sulphate Ash | Found not ponderable | Nominal Value: ≤ 0,5% |
| Heavy Metals | > 50 ppm | ≤ 50 ppm |
| Loss of Drying | Found not ponderable | ≤ 2 |
| Purity (TLC) | no side spots | no side spots |
| Percentage of Side Products | > 0.2 mole | ≤ 10 mole |
| Content | 98,7 | 95—105 |

Utilization of NPT 15459 to produce NPT 15392

The reaction scheme described below illustrated the use of NPT 15459 in the synthesis of NPT 15392:

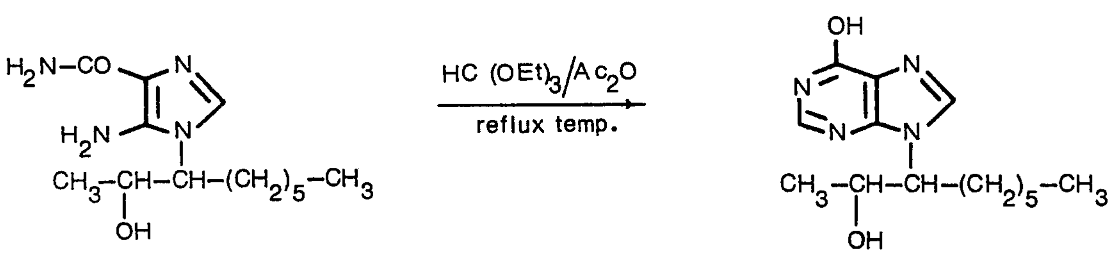


(V) $C_{13}H_{24}N_4O_2$ 268.35

(VI) $C_{14}H_{22}N_4O_2$ 278.35

The homologues of NPT 15392 can be prepared in the same manner from the corresponding homologues of NPT 15459. In place of ethyl orthoformate there can be employed the other lower alkyl orthoformates, e.g. the methyl, propyl, and butyl orthoformates. In forming NPT 15392 the acetic anhydride forms the acetate of NPT 15392 and this is then hydrolized with alkali, e.g. sodium hydroxide.

In place of an orthoformic acid ester formic acid can also be used.

Example 2

Utilization of NPT 15459 to produce NPT 15392 (Erythro-9-(2-hydroxy-3-nonyl)-hypoxanthine)

415 g of erythro-3-(2-hydroxy-3-nonyl)-4-amino-imidazole-5-carboxamide (V) (1.55 moles) are suspended in 383 ml of orthoformic acid triethylester. 220 ml of acetic acid anhydride are added with stirring. The suspension that is obtained is heated with stirring up to 100—105°C. An exothermic reaction ensues with liberation of ethanol. The liberated ethanol is separated by distillation. The temperature of the reaction mixture is increased slowly up to 130—140°C. Stirring is continued for about 3 hours, while the liberated ethanol is continuously separated by distillation. The reaction mixture is concentrated to a viscous syrup, which is stirred into a mixture of 415 ml of 30% aqueous NaOH and 1.68 liter of water. The brown solution is slightly acidified by addition of a solution of 123 ml of acetic acid in 1.5 liter of water. Erythro-9-(2-hydroxy-3-nonyl)-hypoxanthine (VI) crystallizes, is filtered by suction, washed with water and dried in vacuo at 60°C.

Yield: 350 g corresponding to 81.4% of theory

Melting point (after repated recrystallization from aqueous ethanol): 200—201°C
Content by titration: 99.4%
Content of threo isomer: 0.9%

Utilization of NPT 15459 to produce novel derivatives of NPT 15392

NPT 15459 and its homologues possess the ability to react with a variety of reagents which could lead to ring closure and the production of certain purine derivatives. By the judicious choice of reagents, it is possible to produce 2-lower alkyl substituted derivates of NPT 15392 and its homologues that could not be produced without difficulty by other means.

Compounds of general formula 1 where $R_3$ is methyl can be made by reacting NPT 15459 or its homologues with ethyl orthoacetate rather than ethyl orthoformate. Compounds of general formula 1 where $R_3$ is ethyl can be made in similar manner by reacting NPT 15459 or its homologues with ethyl orthopropionate.

The immunomodulating activity of NPT 15459 was determined. The results are set forth below in Tables 1 and 2.

In Table 1 the effect of NPT 15459 on con A Induced Proliferation is described. The procedure is described in Simon U.S. patent 4,221,909, column 24, line 12 to column 25, line 10.

In Table 1 the effect of NPT 15459 on LPS Induced Proliferation is described.

In Table 2 the SRBC Induced Antibody Formation procedure employed was that shown in Simon EP—A—0036077 page 46.

TABLE 1
Immunomodulating activity NPT 15459
Mitogen Induced Lymphocyte Proliferation

| Concentration (Dosage) | Test | $\overline{X}$ | Percent Change from Control |
|---|---|---|---|
| | | cpm | |
| Control | Con A induced Proliferation | 35,059 | 0 |
| .020 μg/ml | " " | 35,747 | +2 |
| .500 " | " " | 30,920 | −12 |
| 5.0 " | " " | 20,098 | −43[a] |
| 10.0 " | " " | 12,736 | −66[a] |
| Control | LPS Induced Proliferation | 7044 | 0 |
| .020 μg/ml | " " | 6184 | −12 |
| .5 " | " " | 6676 | −6 |
| 5.0 " | " " | 4128 | −42[a] |
| 10.0 " | " " | 2449 | −66[a] |

TABLE 2
Immunomodulating activity NPT 15459
Antibody Formation

| Concentration (Dosage) | Test | $\overline{X}$ | Percent Change from Control |
|---|---|---|---|
| | | PFC/$10^6$ Cells | |
| Saline | SRBC Induced Antibody Formation | 53 | 0 |
| .05 mg/kg | | 157 | +196 |
| .5 " | | 120 | +126 |
| 5.0 " | | 66 | +24 |

**Claims**

1. A process of preparing a compound of general formula 1

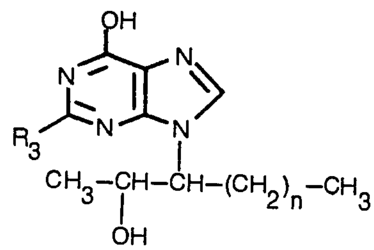

(1)

wherein

$R_3$ is hydrogen or lower alkyl and

n is an integer from 1 to 5, comprising reacting a compound of the general formula 2

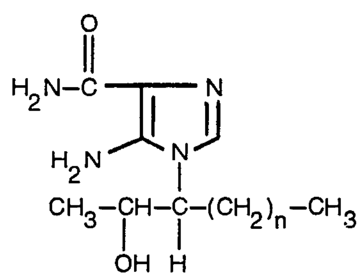

(2)

with a lower alkyl orthoester of a lower fatty acid or with formic acid in the presence of acetic anhydride.

2. A process of preparing a compound of the general formula 3

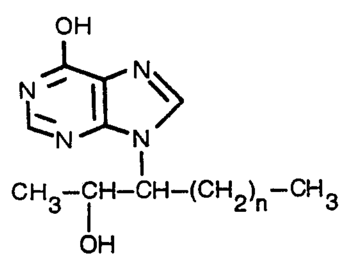

(3)

wherein

n is an integer from 1 to 5, comprising reacting a compound of the general formula 2 of claim 1 with an equimolar amount of a lower alkyl orthoformate or with formic acid in the presence of acetic anhydride.

3. A process according to claim 2 wherein there is employed a lower alkyl orthoformate.

4. A process according to claim 2 wherein there is employed formic acid.

5. A process according to claim 1 wherein $R_3$ is methyl and there is employed a lower alkyl orthoacetate.

6. A process according to claim 5 wherein there is employed ethyl orthoacetate.

7. A process according to claim 1 wherein $R_3$ is ethyl and there is employed a lower alkyl orthopropionate.

8. A process according to claim 7 wherein there is employed ethyl orthopropionate.

9. A process according to any one of claims 1—8 comprising removing the alcohol formed in the reaction and adding alkali after removing the alcohol to neutralize the acetic anhydride.

10. A process according to any one of claims 1—3 and 7, wherein the or each lower alkyl group has 1—4 carbon atoms.

11. A process for preparing erythro-9-(2-hydroxy-3-nonyl)-hypoxanthine, the process comprising the steps of:

reacting 2-amino-2-cyano-acetamide with methyl or ethyl orthoformate to form 2-methoxy or 2-ethoxy-methyl-amino-2-cyanoacetamide;

reacting the 2-methoxy or 2-ethoxy-methylamino-2-cyanoacetamide with erythro-3-amino-2-nonanol to obtain erythro-3-(2-hydroxy-3-nonyl)-4-amino-imidazole-5-carboxamide, and

reacting the erythro-3-(2-hydroxy-3-nonyl)-4-amino-imidazole-5-carboxamide with methyl or ethyl orthoformate.

12. The process of claim 11 wherein the step of reaction the erythro-3-(2-hdyroxy-3-nonyl)-4-amino-imidazole-5-carboxamide with methyl or ethyl orthoformate is conducted in the presence of acetic anhydride.

**Patentansprüche**

1. Verfahren zur Herstellung einer Verbindung der allgemeinen Formel 1

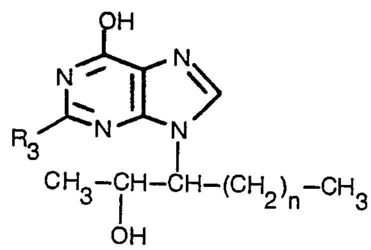

(I)

worin

$R_3$ für Wasserstoff oder Niederalkyl steht und

n eine ganze Zahl von 1 bis 5 ist, wobei man eine Verbindung der allgemeinen Formel 2

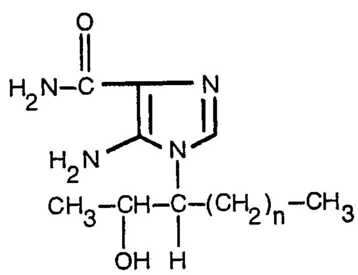

(2)

in Gegenwart von Essigsäureanhydrid mit einem Niederalkylorthoester einer niederen Fettsäure oder mit Ameisensäure umsetzt.

2. Verfahren zur Herstellung einer Verbindung der allgemeinen Formel 3,

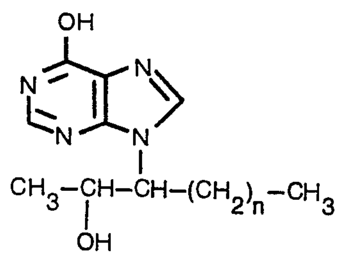

(3)

worin

n eine ganze Zahl von 1 bis 5 ist, wobei man eine Verbindung der allgemeinen Formel 2 nach Anspruch 1 in Gegenwart von Essigsäureanhydrid mit einer äqimolaren Menge eines Orthoameisensäureniederalkylesters oder mit Ameisensäure umsetzt.

3. Verfahren nach Anspruch 2, worin man einen Orthoameisensäureniederalkylester einsetzt.

4. Verfahren nach Anspruch 2, worin man Ameisensäure einsetzt.

5. Verfahren nach Anspruch 1, worin $R_3$ für Methyl steht und man einen Orthoessigsaüreniederalkylester einsetzt.

6. Verfahren nach Anspruch 5, worin man Orthoessigsäureethylester einsetzt.

7. Verfahren nach Anspruch 1, worin $R_3$ für Ethyl steht und man einen Orthopropionsäurenideralkylester einsetzt.

8. Verfahren nach Anspruch 7, worin man Orthopropionsäureethylester einsetzt.

9. Verfahren nach einem der Ansprüche 1—8, wobei man den der Reaktion gebildeten Alkohol entfernt und nach Entfernung des Alkohls zum Neutralisieren des essigsäureanhydrids Alkali zusetzt.

10. Verfahren nach einem der Ansprüche 1—3, 5 und 7, worin die Niederalkylgruppe(n) jeweils 1—4 Kohlenstoffatome aufweisen.

11. Verfahren zur Herstellung von Erythro-9-(2-hydroxy-3-nonyl)-hypoxanthin, wobei man stufenweise:

2-Amino-2-cyanacetamid mit Orthoameisensäuremethyl- oder- ethylester zu 2-Methoxy- oder 2-Ethoxymethylamino-2-cyanacetamid umsetzt,

das 2-Methoxy- oder 2-Ethoxy-methylamino-2-cyanacetamid mit Erythro-3-amino-2-nonanol zu Erythro-3-(2-hydroxy-3-nonyl)-4-amino-imidazol-5-carboxamid und

das Erythro-3-(2-hydroxy-3-nonyl)-4-amino-imidazol-5-carboxamid mit Orthoameisensäuremethyl- oder -ethylester umsetzt.

12. Verfahren nach Anspruch 11, wobei man die Stufe der Umsetzung des Erythro-3-(2-hydroxy-3-nonyl)-4-amino-imidazol-5-carboxamids mit Orthoameisensäuremethyl- oder -ethylester in Gegenwart von Essigsäureanhydrid durchführt.

**Revendications**

1. Procédé de préparation d'un composé de formule générale 1

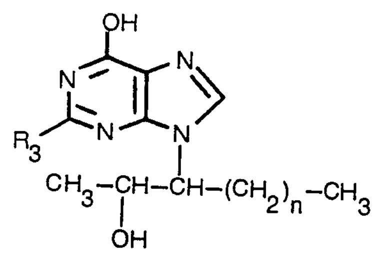

(I)

dans laquelle

$R_3$ est un hydrogène ou un groupe alkyle inférieur et

n est un nombre entier de 1 à 5, comprenant la réaction d'un composé de formule générale

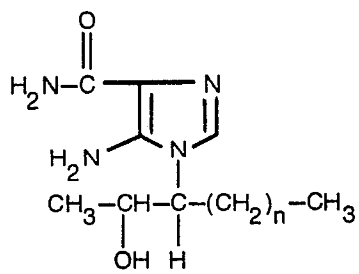

(2)

avec un orthoester d'alkyle inférieur d'un acide gras inférieur ou avec l'acide formique, en présence d'anhydride acétique.

2. Procédé de préparation d'un composé de formule générale 3

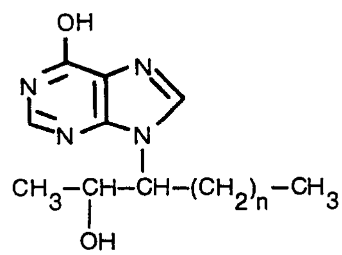

(3)

dans laquelle

n est un nombre der 1 à 5, comprenant la réaction d'un composé de formule générale 2 selon la revendication 1 avec une quantité équimolaire d'un orthoformiate d'alkyle inférieur ou avec l'acide formique, en présence d'anhydride acétique.

3. Procédé selon la revendication 2, dans lequel on emploie un orthoformiate d'alkyle inférieur.

4. Procédé selon la revendication 2, dans lequel on emploie un l'acide formique.

5. Procédé selon la revendication 1, dans lequel $R_3$ est un groupe méthyle et on emploie un orthoacétate d'alkyle inférieur.

6. Procédé selon la revendication 5, dans lequel on emploie l'orthoacétate d'éthyle.

7. Procédé selon la revendication 1, dans lequel $R_3$ est un groupe éthyle et on emploie un orthopropionate d'alkyle inférieur.

8. Procédé selon la revendication 7, dans lequel on emploie l'orthopropionate d'éthyle.

9. Procédé selon l'une quelconque des revendications 1—8, comprenant l'élimination de l'acool formé dans la réaction et l'addition d'une base, après l'élimination de l'alcool, pour neutraliser l'anhydride acétique.

10. Procédé selon l'une quelconque des revendications 1—3, 5, 7, dans lequel le groupe ou chaque groupe alkyle inférieur comporte 1—4 atomes de carbone.

11. Procédé de préparation d'érythro-9-(2-hydroxy-3-nonyl)-hypoxanthine, le procédé comprenant les étapes qui consistent à :

faire réagir le 2-amino-2-cyanoacétamide avec l'orthoformiate de méthyle ou d'éthyle pour former le 2-méthoxy- ou 2-éthoxy-méthylamino-2-cyanoacétamide;

faire réagir le 2-méthoxy- ou 2-éthoxy-méthylamino-2-cyanoacétamide avec l'érythro-3-amino-2-nonanol pour obtenir l'érythro-3-(2-hydroxy-3-nonyl)-4-aminoimidazole-5-carboxamide, et

faire reagir l'érythro-3-(2-hydroxy-3-nonyl)-4-aminoimidazole-5-carboxamide avec l'orthoformiate de méthyle ou d'éthyle.

12. Procédé selon la revendication 11, dans lequel l'étape de réaction de l'érythro-3-(2-hydroxy-3-nonyl)-4-aminoimidazole-5-carboxamide avec l'orthoformiate de méthyle ou d'éthyle est réalisée en présence d'anhydride acétique.

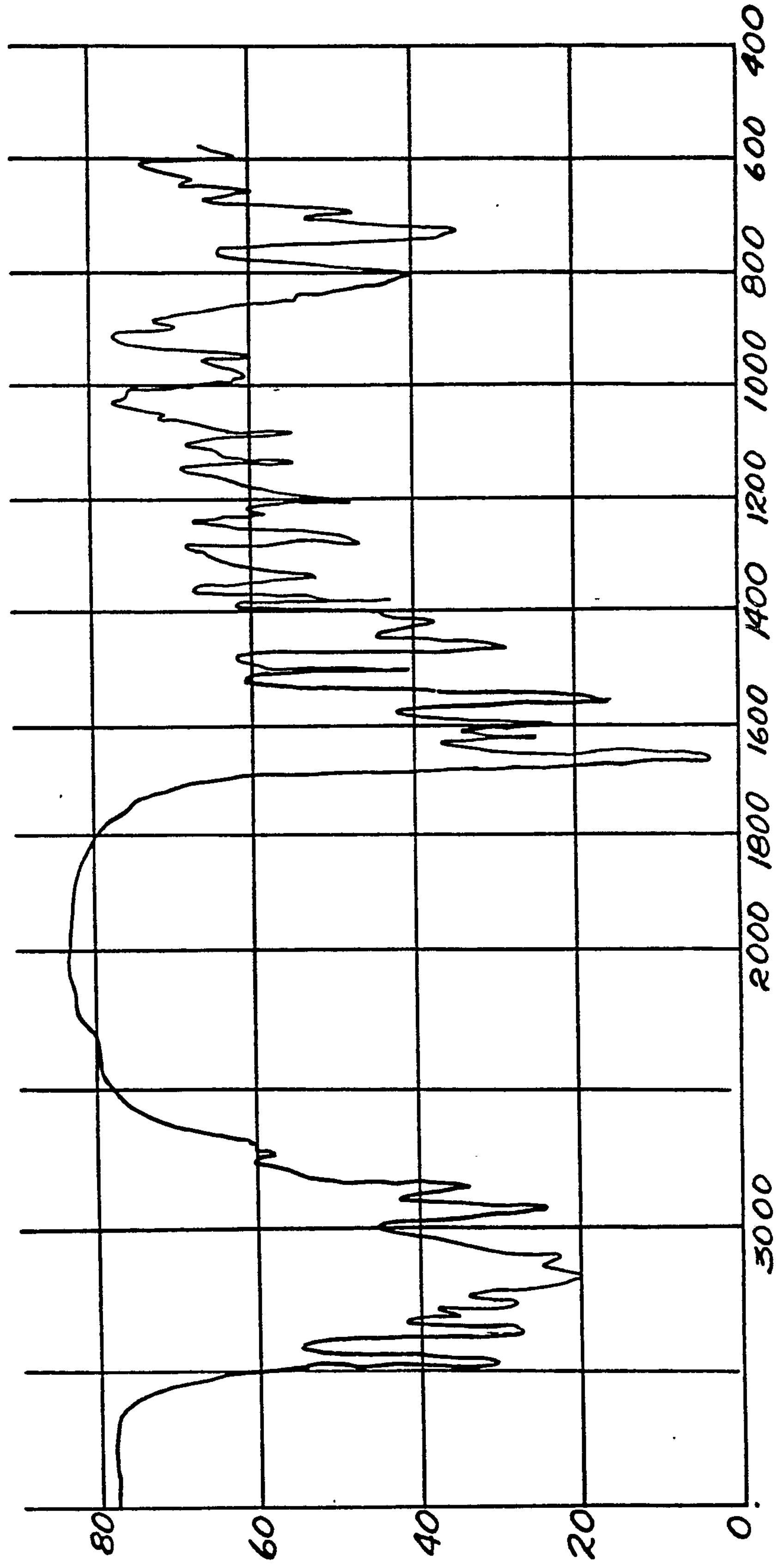


Fig. 1.

Fig. 2.